# EUROPÄISCHE PATENTSCHRIFT

(11) **EP 2 265 296 B1**
(45) Veröffentlichungstag und Bekanntmachung des Hinweises auf die Patenterteilung: **04.07.2012**
(21) Anmeldenummer: 09723136.9
(22) Anmeldetag: 16.03.2009
(51) Int. Cl.: A61L 29/08, A61L 31/10, C08G 18/10, C08G 18/12, C08G 18/28, C08G 18/44, C08G 18/66, C08G 18/72, C08G 18/75, C09D 175/02, C09D 175/04

(54) **MEDIZINISCHE GERÄTE MIT HYDROPHILEN BESCHICHTUNGEN**
MEDICAL DEVICE HAVING HYDROPHILIC COATINGS
APPAREILS MÉDICAUX DOTÉS DE REVÊTEMENTS HYDROPHILES

(30) Priorität: 20.03.2008 EP 08153059
(43) Veröffentlichungstag der Anmeldung: 29.12.2010
(73) Patentinhaber: Bayer MaterialScience AG, 51368 Leverkusen (DE)
(72) Erfinder: KÖCHER, Jürgen, 40764 Langenfeld (DE)
(86) Internationale Anmeldenummer: PCT/EP2009/001900
(87) Internationale Veröffentlichungsnummer: WO 2009/115265

(56) Entgegenhaltungen:
- WO-A-2006/037321
- WO-A-2006/101573
- JP-A- 2002 128 859
- US-A- 5 041 100
- US-A1- 2005 182 188

## Beschreibung

Die Erfindung betrifft medizinische Geräte mit hydrophilen und blutverträglichen Beschichtungen aus Polyurethanharnstoffen. Diese medizinischen Geräte mit verbesserter Oberflächenbeschaffenheit bieten aufgrund einer geringeren Reibung und durch deren Fähigkeit, bei Kontakt mit Blut die Gefahr von Blutgerinnseln zu reduzieren, Vorteile bei der Anwendung.

Die Benutzung von medizinischen Geräten, beispielsweise von Kathetern, kann durch die Ausrüstung mit hydrophilen Oberflächen stark verbessert werden. Das Einsetzen und Verschieben von Urin- oder Blutgefäßkathetern wird dadurch einfacher, dass hydrophile Oberflächen im Kontakt mit Blut oder Urin einen Wasserfilm adsorbieren. Hierdurch wird die Reibung der Katheteroberfläche gegenüber den Gefäßwänden verringert, so dass sich der Katheter leichter einsetzen und bewegen lässt. Auch eine direkte Wässerung der Geräte vor dem Eingriff kann vorgenommen werden, um durch die Bildung eines homogenen Wasserfilms die Reibung zu vermindern. Die betroffenen Patienten haben weniger Schmerzen, und das Risiko von Verletzungen der Gefäßwände wird dadurch reduziert. Darüber hinaus besteht bei der Anwendung von Kathetern immer die Gefahr, dass sich Blutgerinnsel bilden.

Katheter mit hydrophil ausgerüsteten Oberflächen sind aus dem Stand der Technik an sich bekannt.

So beschreibt beispielsweise die WO 99/38545 A1 Katheter, die in einer ersten Ausführungsform aus einer Grundbeschichtung und einer gleitfähigen hydrophilen Beschichtung bestehen. Ferner wird in diesem Stand der Technik auch eine Ausführungsform beschrieben, in welcher nur eine gleitfähige Beschichtung, d.h. ein Beschichtungssystem ohne Grundbeschichtung, verwendet wird. In diesem Fall wird eine gleitfähige Beschichtung aus einem Polyurethan verwendet.

Aus der WO 2006/037321 A1 sind medizinische Geräte mit einer angefeuchteten hydrophilen Oberfläche bekannt, welche die Gleiteigenschaften des Gerätes verbessern soll. Die Oberfläche wird gebildet durch eine Beschichtungszusammensetzung mit einem hydrophilen Polymer und einem Befeuchtungsmittel, umfassend Wasser und mindestens ein Gleitmittel.

Aus der US 2003/0203991 A1 sind hydrophile Beschichtungsmaterialien bekannt, die auf Mischungen von hydrophoben mit hydrophilen Polymeren basieren. Entsprechende Beschichtungszusammensetzungen für medizinische Geräte umfassen (a) eine wässrige polymere Matrix; (b) ein hydrophiles Polymer; (c) ein kolloidales Metalloxid und (d) ein Vernetzer. Die erforderliche Hydrophilie der Beschichtung gemäß US 2003/0203991 A1 wird durch das Polymer (b) erreicht, welches in die entsprechende polymere Matrix eingearbeitet ist. Als polymere Matrix, nicht jedoch als hydrophiles Polymer, werden unter anderem auch Polyurethandispersionen verwendet.

Mischungen aus Polyurethan und Polyvinylpyrrolidon als der die Hydrophilie bewirkende Bestandteil werden ferner in US 5,061,424 beschrieben. Darüber hinaus beschreiben US 5,041,100 und US 2005/054774 A1 jeweils polyurethanhaltige Beschichtungszusammensetzungen mit Polyethylenoxid (US 5,041,100) bzw. Acrylaten (US 2005/054774) als die die Hydrophilie bewirkenden Bestandteile.

US 2006/040253 A1 beschreibt hydrophile Beschichtung von medizinischen Geräten zur Verbesserung der Gleiteigenschaften, wobei die Zusammensetzung zumindest ein wasserlösliches gleitfähiges Polymer und ein unlösliches Polymer aufweist. Das wasserlösliche gleitfähige Polymer wird unter anderem ausgewählt aus der Gruppe, bestehend aus Polyethylenoxid, Polypropylenoxid, Polyethylvinylalkohol, Polyethylvinylacetat und Polyvinylpyrrolidon, während das unlösliches Polymer unter anderem durch Polyurethane, Polyesterurethane und Polyetherunrethane gebildet wird.

Aliphatische Polyetherpolyurethane für hydrophile Beschichtungen sind ebenfalls kommerziell erhältlich, so zum Beispiel das Tecogel^{®} (Thermedics Polymer Products) oder das Hydroslip^{®} (CardioTech International Inc.).

Sowohl die in der Literatur beschriebenen Mischungen als auch die kommerziell erhältlichen Polyetherpolyurethane weisen verschiedene Nachteile auf. Die Mischungen sind Mehrkomponentensysteme und sind daher aufwändig in der Herstellung, insbesondere auch solche Systeme, die durch kovalente Verknüpfung zweier Polymere aufgebaut werden (vgl. US 2003/0203991 A1).

Als Alternative zu Beschichtungssystemen aus organischer Lösung sind auch in wässrigem Medium dispergierte Polyurethane denkbar. Ein Nachteil solcher wässriger Dispersionen ist, dass durch die Größe der dispergierten Partikel die Beschichtungen relativ rau sind. Auch sind Filme aus Dispersionspolymeren üblicherweise nicht ausreichend stabil. Daher gibt es nach wie vor Bedarf für hydrophile Beschichtungssysteme, welche ausgehend von Polyurethan hergestellt werden.

In diesem Kontext empfiehlt die US 5,589,563 die Verwendung von Beschichtungen mit oberflächenmodifizierten Endgruppen für im Bereich der Biomedizin verwendete Polymere, hergestellt und die polymeren Beschichtungen umfassen unterschiedliche Endgruppen, welche ausgewählt werden aus Aminen, fluorierten Alkanolen, Polydimethylsiloxanen und aminterminierten Polyethylenoxiden. Diese Polymere weisen jedoch als Beschichtung für medizinische Geräte keine zufriedenstellenden Eigenschaften, insbesondere im Hinblick auf die erforderliche Hydrophilie, auf.

Die Aufgabe der vorliegenden Erfindung ist somit zunächst die Bereitstellung von medizinischen Geräten mit hydrophilen Oberflächen. Da diese Oberflächen häufig im Blutkontakt eingesetzt werden, sollen die Oberflächen dieser Materialien auch eine gute Blutverträglichkeit besitzen und insbesondere die Gefahr der Bildung von Blutgerinnseln reduzieren.

Gegenstand dieser Erfindung ist Verwendung einer Lösung, enthaltend mindestens einen Polyurethanharnstoff, wobei der Polyurethanharnstoff mit einer Copolymereinheit aus Polyethylenoxid und Polypropylenoxid terminiert ist, zur Herstellung einer Beschichtung für ein medizinisches Gerät,

Die medizinischen Geräte umfassen mindestens eine Beschichtung, enthaltend mindestens einen Polyurethanharnstoff, der mit einer Copolymereinheit aus Polyethylenoxid und Polypropylenoxid terminiert ist

Erfindungsgemäß wurde herausgefunden, dass sich Zusammensetzungen aus diesen speziellen Polyurethanhamstoffen in Lösungen hervorragend zur Herstellung von Beschichtungen auf medizinischen Geräten eignen, diese mit einer hervorragenden gleitfähigen Beschichtung versehen und gleichzeitig die Gefahr der Bildung von Blutgerinnseln während der Behandlung mit dem medizinischen Gerät reduzieren.

Polyurethanharnstoffe im Sinne der vorliegenden Erfindung sind polymere Verbindungen, die
(a) mindestens zwei Urethangruppen enthaltende Wiederholungseinheiten der folgenden allgemeinen Struktur 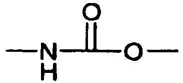 und
   mindestens eine Harnstoffgruppen enthaltende Wiederholungseinheit 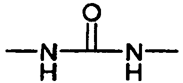 aufweisen.

Die erfindungsgemäß zu verwendenden Beschichtungszusammensetzungen basieren auf Polyurethanhanstoffen, welche im Wesentlichen keine ionische Modifizierung aufweisen. Hierunter wird im Rahmen der vorliegenden Erfindung verstanden, dass die erfindungsgemäß zu verwendenden Polyurethanharnstoffe im Wesentlichen keine ionischen Gruppen, wie insbesondere keine Sulfonat-, Carboxylat-, Phosphat- und Phosphonatgruppen, aufweisen.

Unter dem Begriff "im Wesentlichen keine ionische Gruppen" wird im Rahmen der vorliegenden Erfindung verstanden, dass die resultierenden Beschichtung des Polyurethanharnstoffs ionische Gruppen mit einem Anteil von im Allgemeinen höchstens 2,50 Gew.-%, insbesondere höchstens 2,00 Gew.-%, vorzugsweise höchstens 1,50 Gew.-%, besonders bevorzugt höchstens 1,00 Gew.-%, speziell höchstens 0,50 Gew.-%, noch spezieller keine ionische Gruppen aufweist. Damit ist insbesondere bevorzugt, dass der Polyurethanharnstoff keine ionische Gruppen aufweist, da hohe Konzentration an Ionen in organischer Lösung dazu führen, dass das Polymer nicht mehr ausreichend löslich ist und somit keine stabilen Lösungen erhalten werden können. Falls das erfindungsgemäß verwendete Polyurethan ionische Gruppen aufweist, so handelt es sich bevorzugt um Carboxylate.

Die erfindungsgemäß zur Beschichtung der medizinischen Geräte vorgesehenen Polyurethanharnstoffe sind bevorzugt im Wesentlichen lineare Moleküle, können jedoch auch verzweigt sein, was jedoch weniger bevorzugt ist. Unter im Wesentlichen linearen Molekülen versteht man leicht anvernetzte Systeme, die ein Polycarbonatpolyol mit einer mittleren Hydroxylfunktionalität von vorzugsweise 1,7 bis 2,3, insbesondere 1,8 bis 2,2, besonders bevorzugt 1,9 bis 2,1, aufweisen.

Das zahlenmittlere Molekulargewicht der erfindungsgemäß bevorzugt verwendeten Polyurethanharnstoffe beträgt vorzugsweise 1000 bis 200000, besonders bevorzugt von 5000 bis 100000. Dabei wird das zahlenmittlere Molekulargewicht gegen Polystyrol als Standard in Dimethylacetamid bei 30 °C gemessen.

### Polyurethanhamstoffe

Im Folgenden werden die erfindungsgemäß zu verwendenden Beschichtungssysteme auf Basis von Polyurethanharnstoffen näher beschrieben.

Die erfindungsgemäß in den Beschichtungen von medizinischen Geräten verwendeten Polyurethanharnstoffe werden durch Umsetzung mindestens einer Polycarbonatpolyolkomponente, mindestens einer Polyisoyanatkomponente, mindestens einem Polyoxyalkylenether, mindestens einem Diamin und/oder einem Aminoalkohol und gegebenenfalls einer Polyolkomponente gebildet.

### (a) Polycarbonatpolyol

Die Zusammensetzung der erfindungsgemäß vorgesehene Beschichtung eines Polyurethanharnstoffes weist Einheiten auf, welche auf mindestens ein Polycarbonatpolyol zurückgehen.

Die dabei in einer Ausführungsform verwendete Polycarbonatpolyolkomponente ist ein Hydroxylgruppen enthaltendes Polycarbonat.

Grundsätzlich geeignet für das Einführen von Einheiten auf Basis eines Hydroxylgruppen enthaltenden Polycarbonats sind Polyhydroxyverbindungen mit einer mittleren Hydroxylfunktionalität von 1,7 bis 2,3, vorzugsweise von 1,8 bis 2,2, besonders bevorzugt von 1,9 bis 2,1.

Als Hydroxylgruppen aufweisende Polycarbonate kommen Polycarbonate des durch OH-Zahl bestimmten Molekulargewichts von vorzugsweise 400 bis 6000 g/mol, besonders bevorzugt 500 bis 5000 g/mol, insbesondere von 600 bis 3000 g/mol in Frage, die beispielsweise durch Reaktion von Kohlensäurederivaten, wie Diphenylcarbonat, Dimethylcarbonat oder Phosgen, mit Polyolen, vorzugsweise Diolen, erhältlich sind. Als derartige Diole kommen beispielsweise Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentan-1,3-diol, Di-, Tri- oder Tetraethylenglykol. Dipropylenglykol, Polypropylenglykole, Dibutylenglykol, Polybutylenglykole, Bisphenol A, Tetrabrombisphenol A aber auch Lacton-modifizierte Diole in Frage.

Bevorzugt enthält die Diolkomponente 40 bis 100 Gew.-% Hexandiol, bevorzugt 1,6-Hexandiol und/oder Hexandiol-Derivate, vorzugsweise solche, die neben endständigen OH-Gruppen Ether- oder Estergruppen aufweisen, z.B. Produkte, die durch Umsetzung von 1 Mol Hexandiol mit mindestens 1 Mol, bevorzugt 1 bis 2 Mol Caprolacton oder durch Veretherung von Hexandiol mit sich selbst zum Di- oder Trihexylenglykol erhalten wurden. Auch Polyether-Polycarbonatdiole können eingesetzt werden. Die Hydroxylpolycarbonate sollten im wesentlichen linear sein. Sie können jedoch gegebenenfalls durch den Einbau polyfunktioneller Komponenten, insbesondere niedermolekularer Polyole, leicht verzweigt werden. Hierzu eignen sich beispielsweise Glycerin, Trimethylolpropan, Hexantriol-1,2,6, Butantriol-1,2,4, Trimethylolpropan, Pentaerythrit, Chinit, Mannit, Sorbit, Methylglykosid oder 1,3,4,6-Dianhydrohexite. Bevorzugt sind solche Polycarbonate auf Basis von Hexandiol-1,6, sowie modifizierend wirkenden Co-Diolen wie z. B. Butandiol-1,4 oder auch von ε-Caprolacton. Weitere bevorzugte Polycarbonatdiole sind solche auf Basis von Mischungen aus Hexandiol-1,6 und Butandiol-1,4.

Das Polycarbonat ist vorzugsweise im Wesentlichen linear ausgebildet und weist nur eine geringfügige dreidimensionale Vernetzung auf, so dass Polyurethane gebildet werden, welche die zuvor genannte Spezifikation aufweisen.

### (b) Polyisocyanat

Die Zusammensetzung der erfindungsgemäß vorgesehene Beschichtung eines Polyurethanharnstoffes weist Einheiten auf, welche auf mindestens ein Polyisocyanat zurückgehen.

Als Polyisocyanate (b) können alle dem Fachmann bekannten aromatischen, araliphatischen, aliphatischen und cycloaliphatischen Isocyanate einer mittleren NCO-Funktionalität ≥ 1, bevorzugt ≥ 2 einzeln oder in beliebigen Mischungen untereinander eingesetzt werden, wobei es unerheblich ist, ob diese nach Phosgen- oder phosgen-freien Verfahren hergestellt wurden. Diese können auch Iminooxadiazindion-, Isocyanurat-, Uretdion-, Urethan-, Allophanat-, Biuret-, Harnstoff-, Oxadiazintrion, Oxazolidinon-, Acylharnstoff- und/oder Carbodiimid-Strukturen aufweisen. Die Polyisocyanate können einzeln oder in beliebigen Mischungen untereinander eingesetzt werden.

Bevorzugt werden Isocyanate aus der Reihe der aliphatischen oder cycloaliphatischen Vertreter eingesetzt, wobei diese ein Kohlenstoffgrundgerüst (ohne die enthaltenen NCO-Gruppen) von 3 bis 30, bevorzugt 4 bis 20 Kohlenstoffatomen aufweisen.

Besonders bevorzugte Verbindungen der Komponente (b) entsprechen der vorstehend genannten Art mit aliphatisch und/oder cycloaliphatisch gebundene NCO-Gruppen wie beispielsweise Bis-(isocyanatoalkyl)ether, Bis- und Tris-(isocyanatoalkyl)benzole, -toluole, sowie -xylole, Propandiisocyanate, Butandiisocyanate, Pentandiisocyanate, Hexandiisocyanate (z.B. Hexamethylendiisocyanat, HDI), Heptandiisocyanate, Octandiisocyanate, Nonandiisocyanate (z.B. Trimethyl-HDI (TMDI) in der Regel als Gemisch der 2,4,4- und 2,2,4-Isomeren), Nonantriisocyanate (z.B. 4-Isocyanatomethyl-1,8-octandiisocyanat), Dekandiisocyanate, Dekantriisocyanate, Undekandiisocyanate, Undekantriisocyanate, Dodecandiisocyanate, Dodekantriisocyanate, 1,3- sowie 1,4-Bis-(isocyanatomethyl)cyclohexane (H₆XDI), 3-Isocyanatomethyl-3,5,5-trimethylcyclohexylisocyanat (Isophorondiisocyanat, IPDI), Bis-(4-isocyanatocyclohexyl)methan (H₁₂MDI) oder Bis-(isocyanatomethyl)norbornan (NBDI).

Ganz besonders bevorzugte Verbindungen der Komponente (b) sind Hexamethylendiisocyanat (HDI), Trimethyl-HDI (TMDI), 2-Methylpentan-1,5-diisocyanat (MPDI), Isophorondiisocyanat (IPDI), 1,3- sowie 1,4-Bis(isocyanatomethyl)cyclohexan (H₆XDI), Bis(isocyanatomethyl)norbornan (NBDI), 3(4)-Isocyanatomethyl-1-methyl-cyclohexylisocyanat (IMCI) und/oder 4,4'-Bis(isocyanatocyclohexyl)methan (H₁₂MDI) oder Gemische dieser Isocyanate. Weitere Beispiele sind Derivate aus den vorstehenden Diisocyanaten mit Uretdion-, Isocyanurat-, Urethan-, Allophanat-, Biuret-, Iminooxadiazindion-und/oder Oxadiazintrionstruktur mit mehr als zwei NCO-Gruppen.

Die Menge an Bestandteil (b) in der erfindungsgemäßen Beschichtung beträgt vorzugsweise 1,0 bis 3,5 mol, besonders bevorzugt 1,0 bis 3,3 mol, insbesondere 1,0 bis 3,0 mol, jeweils bezogen auf den Bestandteil (a) der erfindungsgemäß zu verwendenden Beschichtung.

### (c) Diamin oder Aminoalkohol

Die Zusammensetzung der erfindungsgemäß vorgesehenen Beschichtung eines Polyurethanharnstoffes weist Einheiten auf, welche auf mindestens ein Diamin oder ein Aminoalkohol zurückgehen und als sogenannte Kettenverlängerer (c) dienen.

Solche Kettenverlängerer sind beispielsweise Di- oder Polyamine sowie Hydrazide, z.B. Hydrazin, Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 1,3- und 1,4-Xylylendiamin, α,α,α',α'-Tetramethyl-1,3- und -1,4-xylylendiamin und 4,4'-Diaminodicyclohexylmethan, Dimethylethylendiamin, Hydrazin, Adipinsäuredihydrazid, 1,4-Bis(aminomethyl)cyclohexan, 4,4'-Diamino-3,3'-dimethyldicyclohexylmethan und andere (C₁ - C₄)-Di- und Tetraalkyldicyclo-hexylmethane, z. B. 4,4'-Diamino-3,5-diethyl-3',5'-diisopropyldicyclohexylmethan.

Als Diamine oder Aminoalkohole kommen im Allgemeinen niedermolekulare Diamine oder Aminoalkohole in Betracht, die aktiven Wasserstoff mit gegenüber NCO-Gruppen unterschiedlicher Reaktivität enthalten, wie Verbindungen, die neben einer primären Aminogruppe auch sekundäre Aminogruppen oder neben einer Aminogruppe (primär oder sekundär) auch OH-Gruppen aufweisen. Beispiele hierfür sind primäre und sekundäre Amine, wie 3-Amino-1-Methylaminopropan, 3-Amino-1-Ethylaminopropan, 3-Amino-1-cyclohexylaminopropan, 3-Amino-1- Methylaminobutan, weiterhin Aminoalkohole, wie N-Aminoethylethanolamin, Ethanolamin, 3-Aminopropanol, Neopentanolamin und besonders bevorzugt Diethanolamin.

Der Bestandteil (c) der erfindungsgemäß zu verwendenden Beschichtungszusammensetzung kann bei deren Herstellung als Kettenverlängerer eingesetzt werden.

Die Menge an Bestandteil (c) in der erfindungsgemäß zu verwendenden Beschichtungszusammensetzung beträgt vorzugsweise 0,1 bis 1,5 mol, besonders bevorzugt 0,2 bis 1,3 mol, insbesondere 0,3 bis 1,2 mol, jeweils bezogen auf den Bestandteil (a) der erfindungsgemäß zu verwendenden Beschichtungszusammensetzung.

### (d) Polyoxyalkylenether

Der in der vorliegenden Erfindung verwendete Polyurethanharnstoff weist Einheiten auf, welche auf ein Copolymer aus Polyethylenoxid und Polypropylenoxid zurückgehen. Diese Copolymereinheiten liegen als Endgruppen in dem Polyurethanharnstoff vor und bewirken eine Hydrophilierung der erfindungsgemäßen Beschichtungszusammensetzung.

Nichtionisch hydrophilierende Verbindungen (d) sind beispielsweise einwertige, im statistischen Mittel 5 bis 70, bevorzugt 7 bis 55 Ethylenoxideinheiten pro Molekül aufweisende Polyalkylenoxidpolyetheralkohole, wie sie in an sich bekannter Weise durch Alkoxylierung geeigneter Startermoleküle zugänglich sind (z.B. in Ullmanns Enzyklopädie der technischen Chemie, 4. Auflage, Band 19, Verlag Chemie, Weinheim S. 31-38).

Geeignete Startermoleküle sind beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole. Besonders bevorzugt wird Diethylenglykolmonobutylether als Startermolekül verwendet.

Die Alkylenoxide Ethylenoxid und Propylenoxid können in beliebiger Reihenfolge oder auch im Gemisch bei der Alkoxylierungsreaktion eingesetzt werden können.

Bei den Polyalkylenoxidpolyetheralkoholen handelt es sich um gemischte Polyalkylenoxidpolyether aus Ethylenoxid und Propylenoxid, deren Alkylenoxideinheiten vorzugsweise zu mindestens 30 mol-%, besonders bevorzugt zu mindestens 40 mol-% aus Ethylenoxideinheiten bestehen. Bevorzugte nichtionische Verbindungen sind monofunktionelle gemischte Polyalkylenoxidpolyether, die mindestens 40 mol-% Ethylenoxid-und maximal 60 mol-% Propylenoxideinheiten aufweisen.

Das mittlere Molgewicht des Polyoxyalkylenethers beträgt vorzugsweise 500 g/mol bis 5000 g/mol, besonders bevorzugt 1000 g/mol bis 4000 g/mol, insbesondere 1000 bis 3000 g/mol.

Die Menge an Bestandteil (d) in der erfindungsgemäß zu verwendenden Beschichtungszusammensetzung beträgt vorzugsweise 0,01 bis 0,5 mol, besonders bevorzugt 0,02 bis 0,4 mol, insbesondere 0,04 bis 0,3 mol, jeweils bezogen auf den Bestandteil (a) der erfindungsgemäß zu verwendenden Beschichtungszusammensetzung.

Erfindungsgemäß konnte gezeigt werden, dass sich die Polyurethanharnstoffe mit Endgruppen, die auf gemischten Polyoxyalkylenether aus Polyethylenoxid und Polypropylenoxid basieren, insbesondere dazu eignen, Beschichtungen mit einer hohen Hydrophilie zu erzeugen. Wie weiter unten im Vergleich zu Polyurethanharnstoffen, welche nur durch Polyethylenoxid terminiert sind, gezeigt wird, bewirken die erfindungsgemäßen Beschichtungen einen deutlich geringen Kontaktwinkel und sind somit hydrophiler ausgebildet.

### (e) Polyole

In einer weiteren Ausführungsform umfasst die Zusammensetzung der erfindungsgemäß vorgesehene Beschichtung eines Polyurethanharnstoffes zusätzlich Einheiten, welche auf mindestens ein Polyol zurückgehen.

Die zum Aufbau der Polyurethanharnstoffe eingesetzten niedermolekularen Polyole (e) bewirken in der Regel eine Versteifung und/oder eine Verzweigung der Polymerkette. Das Molekulargewicht beträgt vorzugsweise 62 bis 500 g/mol, besonders bevorzugt 62 bis 400 g/mol, insbesondere 62 bis 200 g/mol.

Geeignete Polyole können aliphatische, alicyclische oder aromatische Gruppen enthalten. Genannt seien hier beispielsweise die niedermolekularen Polyole mit bis zu etwa 20 Kohlenstoffatomen je Molekül, wie z. B. Ethyenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclo-hexyl)propan), sowie Trimethylolpropan, Glycerin oder Pentaerythrit und Mischungen dieser und gegebenenfalls auch weiterer niedermolekularer Polyole. Auch Esterdiole wie z.B. α-Hydroxybutyl-ε-hydroxy-capronsäureester, ω-Hydroxyhexyl-γ-hydroxybuttersäure-ester, Adipinsäure-(ß-hydroxyethyl)ester oder Terephthalsäure-bis(ß-hydroxyethyl)-ester können verwendet werden.

Die Menge an Bestandteil (e) in der erfindungsgemäß zu verwendenden Beschichtungszusammensetzung beträgt vorzugsweise 0,05 bis 1,0 mol, besonders bevorzugt 0,05 bis 0,5 mol, insbesondere 0,1 bis 0,5 mol, jeweils bezogen auf den Bestandteil (a) der erfindungsgemäß zu verwendenden Beschichtungszusammensetzung.

### (f) Weitere amin- und/oder hvdroxyenthaltende Bausteine (Aufbaukomponente)

Die Umsetzung der isocyanathaltigen Komponente (b) mit den hydroxy oder aminfunktionellen Verbindungen (a), (c), (d) und gegebenenfalls (e) erfolgt üblicherweise unter Einhaltung eines leichten NCO-Überschusses gegenüber den reaktiven Hydroxy- oder Aminverbindungen. In diesem Fall verbleiben am Endpunkt der Reaktion durch Erreichen einer Zielviskosität immer noch Reste an aktivem Isocyanat. Diese Reste müssen blockiert werden, damit nicht eine Reaktion mit großen Polymerketten stattfindet. Eine solche Reaktion führt zur dreidimensionalen Vernetzung und Vergelung des Ansatzes. Die Verarbeitung einer solchen Beschichtungslösung ist nur eingeschränkt oder nicht mehr nicht mehr möglich. Üblicherweise enthalten die Ansätze hohe Mengen an Alkoholen. Diese Alkohole blockieren innerhalb von mehreren Stunden beim Stehen lassen oder beim Rühren des Ansatzes bei Raumtemperatur die noch verbliebenen Isocyanatgruppen.

Will man aber den noch verbleibenden Restisocyanatgehalt schnell blockieren, können die erfindungsgemäß vorgesehenen Polyurethanhamstoff-Beschichtungen auch Monomere (f) enthalten, die sich jeweils an den Kettenenden befinden und diese abschließen.

Diese Bausteine leiten sich zum einen von monofunktionellen, mit NCO-Gruppen reaktiven Verbindungen ab, wie Monoaminen, insbesondere mono-sekundären Aminen oder Monoalkoholen. Genannt seien hier beispielsweise Ethanol, n-Butanol, Ethylenglykolmonobutylether, 2-Ethylhexanol, 1-Octanol, 1-Dodecanol, 1-Hexadecanol, Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin und geeignete substituierte Derivate davon.

Da die Bausteine (f) im Wesentlichen in den erfindungsgemäßen Beschichtungen dazu verwendet werden, den NCO-Überschuss zu vernichten, hängt die erforderliche Menge im Wesentlichen von der Menge des NCO-Überschusses ab und kann nicht allgemein spezifiziert werden.

Bevorzugt wird während der Synthese auf diese Bausteine verzichtet. Noch nicht umgesetztes Isocyanat wird dabei vorzugsweise durch die in sehr großen Konzentrationen enthaltenen Lösungsmittelalkohole zu terminalen Urethanen umgesetzt.

### (g) Weitere Bestandteile

Die erfindungsgemäß vorgesehenen Polyurethanharnstoff-Beschichtungen können darüber hinaus weitere, für den angestrebten Zweck übliche Bestandteile wie Additive und Füllstoffe, enthalten. Ein Beispiel hierfür sind pharmakologische Wirkstoffe, Arzneimittel und Additive, welche die Freisetzung von pharmakologischen Wirkstoffen fördern ("drug-eluting-Additive").

Pharmakologische Wirkstoffe bzw. Arzneimittel, welche in den erfindungsgemäßen Beschichtungen auf den medizinischen Geräten verwendet werden können, sind im Allgemeinen beispielsweise thromboresistente Mittel, antibiotische Mittel, Antitumormittel, Wachtumshormone, Antivirusmittel, antiangiogene Mittel, angiogene Mittel, antimitotische Mittel, entzündungshemmende Mittel, Zellzyklus-regulierende Mittel, genetische Mittel, Hormone, sowie deren Homologe, Derivate, Fragmente, pharmazeutische Salze und Kombinationen davon.

Spezifische Beispiele solcher pharmakologischer Wirkstoffe bzw. Arzneimittel schließen somit thromboresistente (nichtthrombogene) Mittel bzw. andere Mittel zur Unterdrückung einer akuten Thrombose, Stenose oder späten Re-Stenose der Arterien ein, beispielsweise Heparin, Streptokinase, Urokinase, Gewebe-Plasminogen-Aktivator, Anti-Thromboxan-B₂-Mittel; Anti-B-Thromoboglobulin, Prostaglandin-E, Aspirin, Dipyridimol, Anti-Thromboxan-A₂-Mittel, muriner monoklonaler Antikörper 7E3, Triazolopyrimidin, Ciprosten, Hirudin, Ticlopidin, Nicorandil usw. Ein Wachstumsfaktor kann als ebenfalls als ein Arzneimittel benutzt werden, um unterintimale fibromuskülare Hyperplasie an der arteriellen Stenosestelle zu unterdrücken, bzw. es kann jeder beliebige andere Hemmstoff des Zellwachstums an der Stenosestelle benutzt werden.

Der pharmakologische Wirkstoff bzw. das Arzneimittel kann auch aus einem Vasodilatator bestehen, um Vasospasmus entgegen zu wirken, zum Beispiel ein Antispasmusmittel wie Papaverin. Das Arzneimittel kann ein vasoaktives Mittel an sich sein, wie Calciumantagonisten, oder α- und β-adrenergische Agonisten oder Antagonisten. Zusätzlich kann das therapeutische Mittel ein biologisches Haftmittel wie Cyanoacrylat in medizinischer Qualität oder Fibrin, welche beispielsweise für das Ankleben einer Gewebeklappe an die Wand einer Koronararterie verwendet wird, sein.

Das therapeutische Mittel kann ferner ein antineoplastisches Mittel wie 5-Fluorouracil, vorzugsweise mit einem kontrollierenden freisetzenden Träger für das Mittel, sein (z.B. für die Anwendung eines fortdauernden kontrollierten freisetzenden antineoplastischen Mittels an einer Tumorstelle).

Das therapeutische Mittel kann ein Antibiotikum, vorzugsweise in Kombination mit einem kontrollierenden freisetzenden Träger für die fortdauernde Freisetzung aus der Beschichtung eines medizinischen Geräts an einen lokalisierten Infektionsherd innerhalb des Körpers, sein. Ähnlich kann das therapeutische Mittel Steroide für den Zweck des Unterdrückens einer Entzündung in lokalisiertem Gewebe oder aus anderen Gründen enthalten.

Spezifische Beispiele geeigneter Arzneimittel umfassen:
(a) Heparin, Heparinsulfat, Hirudin, Hyaluronsäure, Chondroitinsulfat, Dermatansulfat, Keratansulfat, lytische Mittel, einschließlich Urokinase und Streptokinase, deren Homologe, Analoge, Fragmente, Derivate und pharmazeutische Salze davon;
(b) antibiotischen Mitteln wie Penicilline, Cephalosprine, Vacomycine, Aminoglycoside, Quinolone, Polymxine, Erythromycine; Tertracycline, Chloramphenicole, Clindamycine, Lincomycine, Sulfonamide, deren Homologe, Analoge, Derivate, pharmazeutische Salze und Mischungen davon;
(c) Paclitaxel, Docetaxel, Immunsuppressiva wie Sirolimus oder Everolimus, Alkylierungsmittel einschließlich Mechlorethamin, Chlorambucil, Cyclophosphamid, Melphalan und Ifosfamid; Antimetaboliten einschließlich Methotrexat, 6-Mercaptopurin, 5-Fluorouracil und Cytarabin; Pflanzenalkoide einschließlich Vinblastin; Vincristin und Etoposid; Antibiotika einschließlich Doxorubicin, Daunomycin, Bleomycin und Mitomycin; Nitrosurea einschließlich Carmustin und Lomustin; anorganische Ionen einschließlich Cisplatin; biologische Reaktionsmodifikatoren einschließlich Interferon; angiostatinische Mittel und endostatinische Mittel; Enzyme einschließlich Asparaginase; und Hormone einschließlich Tamoxifen und Flutamid, deren Homologe, Analoge, Fragmente, Derivate, pharmazeutische Salze und Mischungen davon; und
(d) antivrale Mittel wie Amantadin, Rimantadin, Rabavirin, Idoxuridin, Vidarabin, Trifluridin, Acyclovir, Ganciclocir, Zidovudin, Phosphonoformate, Interferone, deren Homologe, Analoge, Fragmente, Derivate, pharmazeutische Salze und Mischungen davon; und
e) entzündungshemmende Mittel wie beispielsweise Ibuprofen, Dexamethason oder Methylprednisolon.

### Beschichtungszusammensetzung

In einer bevorzugten Ausführungsform umfasst die erfindungsgemäß vorgesehene Beschichtungszusammensetzung einen Polyurethanharnstoff, welcher aufgebaut wird aus
a) mindestens einem Polycarbonatpolyol;
b) mindestens einem Polyisocyanat;
c) mindestens einem Diamin oder einem Aminoalkohol; und
d) mindestens einem monofunktionellen Polyoxyalkylenether als Copolymerisat aus Polyethylenoxid und Polypropylenoxid.

In einer weiteren Ausführungsform der vorliegenden Erfindung umfasst die erfindungsgemäß vorgesehene Beschichtungszusammensetzung einen Polyurethanharnstoff, welcher aufgebaut wird aus
a) mindestens einem Polycarbonatpolyol;
b) mindestens einem Polyisocyanat;
c) mindestens einem Diamin oder einem Aminoalkohol;
d) mindestens einem monofunktionellen Polyoxyalkylenether als Copolymerisat aus Polyethylenoxid und Polypropylenoxid; und
e) mindestens einem Polyol.

In einer weiteren Ausführungsform der vorliegenden Erfindung umfasst die erfindungsgemäß vorgesehene Beschichtungszusammensetzung einen Polyurethanharnstoff, welcher aufgebaut wird aus
a) mindestens einem Polycarbonatpolyol;
b) mindestens einem Polyisocyanat;
c) mindestens einem Diamin oder einem Aminoalkohol;
d) mindestens einem monofunktionellen Polyoxyalkylenether als Copolymerisat aus Polyethylenoxid und Polypropylenoxid;
e) mindestens einem Polyol; und
f) mindestens einem amin- oder hydroxylhaltiges Monomer, welches sich an den Polymerkettenenden befindet.

Erfindungsgemäß werden zur Beschichtung der medizinischen Geräte besonders bevorzugt Polyurethanharnstoffe eingesetzt, die aufgebaut werden aus
a) mindestens einem Polycarbonatpolyol mit einem mittleren Molgewicht zwischen 400 g/mol und 6000 g/mol und einer Hydroxylfunktionalität von 1,7 bis 2,3 oder aus Mischungen solcher Polycarbonatpolyole;
b) mindestens einem aliphatischen, cycloaliphatischen oder aromatischen Polyisocyanat oder Mischungen solcher Polyisocyanate in einer Menge pro Mol des Polycarbonatpolyols von 1,0 bis 3,5 mol;
c) mindestens einem aliphatischen oder cycloaliphatischen Diamin oder mindestens ein Aminoalkohol als so genannte Kettenverlängerer oder Mischungen solcher Verbindungen in einer Menge pro Mol des Polycarbonatpolyols von 0,1 bis 1,5 mol;
d) mindestens einem monofunktionellen Polyoxyalkylenether aus Ethylenoxid und Propylenoxid oder einer Mischung solcher Polyether mit einem mittleren Molgewicht zwischen 500 g/mol und 5000 g/mol in einer Menge pro Mol des Polycarbonatpolyols von 0,01 bis 0,5 mol;
e) gegebenenfalls einem oder mehreren kurzkettigen aliphatischen Polyolen mit einem Molgewicht zwischen 62 g/mol und 500 g/mol in einer Menge pro Mol des Polycarbonatpolyols von 0,05 bis 1 mol; und
f) gegebenenfalls amin- oder OH haltige Bausteine, die sich an den Polymerkettenenden befinden und diese abschließen.

Erfindungsgemäß weiter bevorzugt werden zur Beschichtung von medizinischen Geräten Polyurethanharnstoffe eingesetzt, die aufgebaut werden aus
a) mindestens einem Polycarbonatpolyol mit einem mittleren Molgewicht zwischen 500 g/mol und 5000 g/mol und einer Hydroxylfunktionalität von 1,8 bis 2,2 oder aus Mischungen solcher Polycarbonatpolyole;
b) mindestens einem aliphatischen, cycloaliphatischen oder aromatischen Polyisocyanat oder Mischungen solcher Polyisocyanate in einer Menge pro Mol des Polycarbonatpolyols von 1,0 bis 3,3 mol;
c) mindestens einem aliphatischen oder cycloaliphatischen Diamin oder mindestens ein Aminoalkohol als so genannte Kettenverlängerer oder Mischungen solcher Verbindungen in einer Menge pro Mol des Polycarbonatpolyols von 0,2 bis 1,3 mol;
d) mindestens einem monofunktionellen Polyoxyalkylenether aus Ethylenoxid und Propylenoxid oder einer Mischung solcher Polyether mit einem mittleren Molgewicht zwischen 1000 g/mol und 4000 g/mol in einer Menge pro Mol des Polycarbonatpolyols von 0,02 bis 0,4 mol;
e) gegebenenfalls einem oder mehreren kurzkettigen aliphatischen Polyolen mit einem Molgewicht zwischen 62 g/mol und 400 g/mol in einer Menge pro Mol des Polycarbonatpolyols von 0,05 bis 0,5 mol; und
f) gegebenenfalls amin- oder OH haltige Bausteine, die sich an den Polymerkettenenden befinden und diese abschließen.

Erfindungsgemäß noch weiter bevorzugt werden zur Beschichtung von Kathetermaterialien Polyurethanharnstoffe eingesetzt, die aufgebaut werden aus
a) mindestens einem Polycarbonatpolyol mit einem mittleren Molgewicht zwischen 600 g/mol und 3000 g/mol und einer Hydroxylfunktionalität von 1,9 bis 2,1 oder aus Mischungen solcher Polycarbonatpolyole;
b) mindestens einem aliphatischen, cycloaliphatischen oder aromatischen Polyisocyanat oder Mischungen solcher Polyisocyanate in einer Menge pro Mol des Polycarbonatpolyols von 1,0 bis 3,0 mol;
c) mindestens einem aliphatischen oder cycloaliphatischen Diamin oder mindestens ein Aminoalkohol als so genannte Kettenverlängerer oder Mischungen solcher Verbindungen in einer Menge pro Mol des Polycarbonatpolyols von 0,3 bis 1,2 mol;
d) mindestens einem monofunktionellen Polyoxyalkylenether aus Ethylenoxid und Propylenoxid oder einer Mischung solcher Polyether mit einem mittleren Molgewicht zwischen 1000 g/mol und 3000 g/mol in einer Menge pro Mol des Polycarbonatpolyols von 0,04 bis 0,3 mol, wobei eine Mischung aus Polyethylenoxid und Polypropylenoxid insbesondere bevorzugt ist; und
e) gegebenenfalls einem oder mehreren kurzkettigen aliphatischen Polyolen mit einem Molgewicht zwischen 62 g/mol und 400 g/mol in einer Menge pro Mol des Polycarbonatpolyols von 0,1 bis 0,5 mol.

### Medizinisches Gerät

Die Bezeichnung "medizinisches Gerät" ist im Rahmen der vorliegenden Erfindung breit zu verstehen. Geeignete, nicht einschränkende Beispiele für medizinische Geräte (einschließlich Instrumente) sind Kontaktlinsen; Kanülen; Kathetern, zum Beispiel urologische Kathetern wie Blasenkathetern oder Harnleiterkathetern; zentralvenöse Katheter; venöse Kathetern oder Einlass- bzw. Auslass-Kathetern; Dilationsballons; Kathetern für die Angioplastie und die Biopsie, Kathetern, die für das Einbringen eines Stents, eines Propf-oder eines Kavafilters verwendet werden; Ballonkathetern oder andere dehnbare medizinische Geräte; Endoskope; Laryngoskope; Trachealgeräte wie Endotrachealschläuche, Atemgeräte und andere Trachealabsauggeräte; bronchoalveolare Spülungskathetern; Kathetern, die bei der Koronarangioplastie verwendet werden; Führungsstäbe, Einführer und Ähnliches; Gefäßpfropfen; Schrittmacherteile; Cochleaimplantate; Zahnimplantatschläuche für die Nahrungszufuhr, Dränageschläuche; und Führungsdrähte.

Darüber hinaus können die erfindungsgemäßen Beschichtungslösungen zur Herstellung von Schutzbeschichtungen, zum Beispiel für Handschuhe, Stents und andere Implantate; extrakorporale (außerkörperliche) Blutschläuche (Blutführungsrohre); Membrane, zum Beispiel für die Dialyse; Blutfilter; Geräte für die Kreislaufunterstützung; Verbandsmaterial für die Wundpflege; Harnbeutel und Stomabeutel verwendet werden. Eingeschlossen sind auch Implantate, die ein medizinisch wirksames Mittel enthalten, wie medizinisch wirksame Mitteln für Stents oder für Ballonoberflächen oder für Kontrazeptiva.

Üblicherweise wird das medizinisches Gerät aus Kathetern, Endoskopen, Laryngoskopen, Endotrachealschläuchen, Ernährungsschläuchen, Führungsstäben, Stents, und andere Implantate gebildet.

Als Substrat der zu beschichtenden Oberfläche kommen viele Materialien in Frage, wie Metalle, Textilien, Keramiken oder Kunststoffe, wobei die Verwendung von Kunststoffen für die Herstellung von medizinischen Geräten bevorzugt ist

Erfindungsgemäß wurde gefunden, dass man medizinische Geräte mit sehr hydrophilen und damit gleitfähigen, blutverträglichen Oberflächen erzeugen kann, indem man zur Beschichtung der medizinischen Geräte wässrige, nichtionisch stabilisierte Polyurethanlösungen der oben beschriebenen Art verwendet. Die oben beschriebenen Beschichtungszusammensetzungen werden vorzugsweise als organische Lösung erhalten und auf die Oberfläche der medizinischen Geräte appliziert.

Dabei sind insbesondere Beschichtungen, die aus einer Mischung von Polycarbonatpolyolen und einem monofunktionellen Polypropylenoxid-Polyethylenoxid-Alkohol aufgebaut sind, bevorzugt.

### Herstellung der Beschichtungen

Im Rahmen der vorliegenden Erfindung ist es insbesondere bevorzugt, dass die Beschichtungen der medizinischen Geräte ausgehend von Lösungen der oben näher beschriebenen Beschichtungszusammensetzung hergestellt werden.

Erfindungsgemäß hat sich herausgestellt, dass sich die resultierenden Beschichtungen auf medizinischen Geräten unterscheiden, je nachdem, ob die oben beschriebene Beschichtungszusammensetzung ausgehend von einer Dispersion oder einer Lösung hergestellt wird.

Dabei weisen die erfindungsgemäßen Beschichtungen auf medizinischen Geräten dann Vorteile auf, wenn sie ausgehend von Lösungen der oben beschriebenen Beschichtungszusammensetzungen erhalten werden.

Ohne an eine Theorie gebunden sein zu wollen, wird erfindungsgemäß jedoch angenommen, dass aufgrund der partikulären Struktur der Polyurethane in wässriger Dispersion die Verfilmung der Polymere nicht vollständig ist. In den Filmen sind die Partikelstrukturen in der Regel noch zu erkennen, beispielsweise durch Atomic Force Microscopy (AFM). Polyurethane aus Lösung liefern glattere Beschichtungen. Aufgrund der innigen Verhakung und Verschlaufung der Polyurethanmoleküle in organischer Lösung sind auch die getrockneten Filme zugfester und gegenüber der Lagerung in Wasser widerstandsfähiger.

In einer weiteren Ausgestaltung betrifft die vorliegende Erfindung daher ein medizinisches Gerät mit mindestens einer hydrophilen Beschichtung, enthaltend mindestens einen Polyurethanharnstoff, der im Wesentlichen keine ionische Modifizierung aufweist, wobei die Beschichtung ausgehend von einer Lösung des Polyurethanharnstoff hergestellt wird.

Die erfindungsgemäßen medizinischen Geräte können dabei mittels verschiedener Verfahren mit den hydrophilen Polyurethanlösungen beschichtet werden. Geeignete Beschichtungstechniken sind hierfür beispielsweise Rakeln, Drucken, Transferbeschichten, Sprühen, Spincoating oder Tauchen.

Die organischen Polyurethanlösungen können nach beliebigen Verfahren hergestellt werden. Als bevorzugt hat sich jedoch folgende Verfahrensweise herausgestellt:

Zur Herstellung der erfindungsgemäß zur Beschichtung zu verwendenden Polyurethanharnstoff-Lösungen werden vorzugsweise das Polycarbonatpolyol, das Polyisocyanat, der monofunktionelle Polyetheralkohol und gegebenenfalls das Polyol in der Schmelze oder in Lösung miteinander umgesetzt, bis alle Hydroxylgruppen verbraucht sind.

Die dabei verwendete Stöchiometrie zwischen den einzelnen an der Umsetzung beteiligten Komponenten ergibt sich aus den zuvor erwähnten Mengenverhältnissen für die erfindungsgemäße Beschichtung.

Die Umsetzung erfolgt bei einer Temperatur von vorzugsweise zwischen 60 und 110 °C, besonders bevorzugt 75 bis 110 °C, insbesondere 90 bis 110 °C, wobei Temperaturen um 110 °C aufgrund der Geschwindigkeit der Umsetzung bevorzugt sind. Höhere Temperaturen können ebenfalls angewendet werden, allerdings besteht dann im Einzelfall und in Abhängigkeit der einzelnen verwendeten Bestandteile das Risiko, dass Zersetzungsprozesse und Verfärbungen in dem entstehenden Polymer auftreten.

Bei dem Prepolymer aus Isocyanat und allen Hydroxylgruppen aufweisenden Komponenten ist die Umsetzung in Schmelze bevorzugt, allerdings besteht die Gefahr, dass es zu hohen Viskositäten der ausreagierten Gemische kommt. In diesen Fällen empfiehlt es sich auch, Lösemittel hinzu zugegeben. Es sollte aber möglichst nicht mehr als ungefähr 50 Gew.-% Lösemittel enthalten sein, da andernfalls die Verdünnung die Reaktionsgeschwindigkeit deutlich verlangsamt.

Bei der Umsetzung von Isocyanat und den Hydroxylgruppen aufweisenden Komponenten kann die Reaktion in der Schmelze in einem Zeitraum von 1 Stunde bis 24 Stunden erfolgen.. Geringe Zugabe von Lösungsmittelmengen führen zu einer Verlangsamung, wobei die Umsetzungszeiträume jedoch in den gleichen Zeiträumen liegen.

Die Reihenfolge der Zugabe bzw. Umsetzung der einzelnen Bestandteile kann von der zuvor angegebenen Reihenfolge abweichen. Dieses kann insbesondere dann von Vorteil sein, wenn die mechanischen Eigenschaften der resultierenden Beschichtungen verändert werden sollen. Wenn man beispielsweise alle Hydroxylgruppen aufweisenden Komponenten gleichzeitig umsetzt, entsteht ein Gemisch aus Hart- und Weichsegmenten. Wenn man beispielsweise das niedermolekulare Polyol nach der Polycarbonytpolyolkomponente zugibt, erhält man definierte Blöcke, was andere Eigenschaften der resultierenden Beschichtungen mit sich bringen kann. Die vorliegende Erfindung ist somit nicht auf eine beliebige Reihenfolge der Zugabe bzw. Umsetzung der einzelnen Bestandteile der Polyurethanbeschichtung beschränkt.

Dann wird weiteres Lösungsmittel zugesetzt und das gegebenenfalls gelöste Kettenverlängerungsdiamin bzw. der gelöste Kettenverlängerungsaminoalkohol (Verbindung (c)) zugegeben.

Die weitere Zugabe des Lösemittel erfolgt vorzugsweise schrittweise, um die Reaktion nicht unnötig zu verlangsamen, was bei einer vollständigen Zugabe der Lösemittelmenge beispielsweise am Anfang der Umsetzung passieren würde. Ferner ist man bei einem hohen Gehalt an Lösemittel zum Beginn der Reaktion an eine im Verhältnis niedrige Temperatur gebunden, welche von der Art des Lösemittels zumindest mitbestimmt wird. Auch dieses führt zu einer Verlangsamung der Reaktion.

Nach Erreichen der Zielviskosität können die noch verbleibenden Reste an NCO durch ein monofunktionelles aliphatisches Amin blockiert werden. Bevorzugt blockiert man die noch verbliebenen Isocanatgruppen durch Umsetzung mit den im Lösungsmittelgemisch enthaltenen Alkoholen.

Als Lösungsmittel für die Herstellung und die Anwendung der erfindungsgemäßen Polyurethanharnstoff-Lösungen kommen alle denkbaren Lösungsmittel und Lösungsmittelgemische wie Dimethylformamid, N-Methylacetamid, Tetramethylharnstoff, N-Methylpyrrolidon, aromatische Lösungsmittel wie Toluol, lineare und cyclische Ester, Ether, Ketone und Alkohole in Frage. Beispiele für Ester und Ketone sind beispielsweise Ethylacetat, Butylacetat, Aceton, γ-Butyrolacton, Methylethylketon und Methylisobutylketon.

Bevorzugt sind Mischungen aus Alkoholen mit Toluol. Beispiele für die Alkohole, die gemeinsam mit dem Toluol verwendet werden, sind Ethanol, n-Propanol, iso-Propanol und 1-Methoxy-2-propanol.

Im Allgemeinen wird in der Umsetzung so viel Lösungsmittel eingesetzt, dass man ungefähr 10 bis 50 gew.-%ige Lösungen, besonders bevorzugt ungefähr 15 bis 45 gew.-%ige Lösungen, besonders bevorzugt ungefähr 20 bis 40 gew.-%ige Lösungen, erhält.

Der Feststoffgehalt der Polyurethanlösungen liegt im Allgemeinen zwischen 5 bis 60 Gew.-%, vorzugsweise 10 bis 40 Gew.-%. Für Beschichtungsversuche können die Polyurethanlösungen beliebig mit Toluol/Alkohol-Gemischen verdünnt werden, um die Dicke der Beschichtung variabel einstellen zu können. Alle Konzentrationen von 1 bis 60 Gew.-% sind möglich, bevorzugt sind Konzentrationen im Bereich 1 bis 40 Gew.-%.

Dabei können beliebige Schichtdicken erreicht werden wie beispielsweise einige 100 nm bis hinauf zu einigen 100 µm, wobei im Rahmen der vorliegenden Erfindung auch höhere und geringere Dicken möglich sind.

Weitere Zusätze wie beispielsweise Antioxidantien oder Pigmente können ebenfalls verwendet werden. Darüber hinaus können gegebenenfalls noch weitere Zusätze wie Griffhilfsmittel, Farbstoffe, Mattierungsmittel, UV-Stabilisatoren, Lichtstabilisatoren, Hydrophobierungsmittel, Hydrophilierungsmittel und/oder Verlaufshilfsmittel verwendet werden.

Ausgehend von diesen Lösungen werden dann durch die zuvor beschriebenen Verfahren die erfindungsgemäß vorgesehenen Beschichtungen hergestellt.

Beschichtet werden können dabei vielerlei Substrate wie Metalle, Textilien, Keramiken und Kunststoffe. Bevorzugt ist die Beschichtung von medizinischen Geräten, die aus Metallen oder Kunststoff gefertigt sind. Als Metalle sind beispielsweise zu nennen: Medizinischer Edelstahl und Nickel-Titan-Legierungen. Es sind viele Polymermaterialien denkbar, aus denen das medizinische Geräte aufgebaut sein kann, beispielsweise Polyamid; Polystyrol; Polycarbonat; Polyether; Polyester; Polyvinylacetat; natürliche und synthetische Kautschuke; Blockcopolymere aus Styrol und ungesättigten Verbindungen wie Ethylen, Butylen und Isopren; Polyethylen oder Copolymeren aus Polyethylen und Polypropylen; Silikon; Polyvinylchlorid (PVC) und Polyurethanen. Zur besseren Haftung des hydrophilen Polyurethane auf dem medizinischen Gerät können als Untergrund vor dem Auftragen dieser hydrophilen Beschichtungsmaterialien noch weitere geeignete Beschichtungen aufgetragen werden.

Die erfindungsgemäßen medizinischen Geräte können durch verschiedene Verfahren mit den hydrophilen Polyurethandispersionen beschichtet werden. Geeignete Beschichtungstechniken sind Rakeln, Drucken, Transferbeschichten, Sprühen, Spincoating oder Tauchen.

Zusätzlich zu den hydrophilen Eigenschaften der Verbesserung der Gleitfähigkeit zeichnen sich die erfindungsgemäß vorgesehenen Beschichtungszusammensetzungen auch durch eine hohe Blutverträglichkeit aus. Hierdurch ist auch ein Arbeiten mit diesen Beschichtungen besonders im Blutkontakt vorteilhaft. Die Materialien zeigen im Vergleich zu Polymeren des Standes der Technik eine reduzierte Gerinnungsneigung im Blutkontakt.

Die Vorteile der erfindungsgemäßen Katheter mit den hydrophilen Polyurethanbeschichtungen werden durch Vergleichsversuche in den folgenden Beispielen dargelegt.

### Beispiele

Die Ermittlung des NCO-Gehaltes der in den Beispielen und Vergleichsbeispielen beschriebenen Harze erfolgte durch Titration gemäß DIN EN ISO 11909.

Die Bestimmung der Festkörpergehalte erfolgte nach DIN-EN ISO 3251. Es wurden 1 g Polyurethandispersion bei 115 °C bis zur Gewichtskonstanz (15-20 min) mittels eines Infrarottrockners getrocknet.

Die Messung der mittleren Teilchengrößen der Polyurethandispersionen erfolgt mit Hilfe des High Performance Particle Sizer (HPPS 3.3) der Firma Malvern Instruments.

Die in % angegebenen Mengenangaben verstehen sich, wenn nicht anders vermerkt, als Gew.-% und beziehen sich auf die erhaltene wässrige Dispersion.

Die Ermittlung des NCO-Gehaltes der in den Beispielen und Vergleichsbeispielen beschriebenen Harze erfolgte durch Titration gemäß DIN EN ISO 11909.

Die Bestimmung der Festkörpergehalte erfolgte nach DIN-EN ISO 3251. Es wurden 1 g Polyurethandispersion bei 115 °C bis zur Gewichtskonstanz (15-20 min) mittels eines Infrarottrockners getrocknet.

Die Messung der mittleren Teilchengrößen der Polyurethandispersionen erfolgt mit Hilfe des High Performance Particle Sizer (HPPS 3.3) der Firma Malvern Instruments.

Die in % angegebenen Mengenangaben verstehen sich, wenn nicht anders vermerkt, als Gew.-% und beziehen sich auf die erhaltene wässrige Dispersion.

Viskositätsmessungen wurden mit dem Physics MCR 51 Rheometer der Firma Anton Paar GmbH, Ostfildern, Deutschland, durchgeführt.

### Verwendete Substanzen und Abkürzungen:

| | |
|---|---|
| Desmophen C2200: | Polycarbonatpolyol, OH-Zahl 56 mg KOH/g, zahlenmittleres Molekulargewicht 2000 g/mol (Bayer MaterialScience AG, Leverkusen, DE) |
| Desmophen C1200: | Polycarbonatpolyol, OH-Zahl 56 mg KOH/g, zahlenmittleres Molekulargewicht 2000 g/mol (Bayer MaterialScience AG, Leverkusen, DE) |
| Desmophen XP 2613 | Polycarbonatpolycl, OH-Zahl 56 mg KOH/g, zahlenmittleres Molekulargewicht 2000 g/mol (Bayer MaterialScience AG, Leverkusen, DE) |
| PolyTHF^{®} 2000: | Polytetramethylenglykolpolyol, OH-Zahl 56 mg KOH/g, zahlenmittleres Molekulargewicht 2000 g/mol (BASF AG, Ludwigshafen, DE) |
| Polyether LB 25: | (monofunktioneller Polyether auf Ethylenoxid-/Propylenoxidbasis zahlenmittleres Molekulargewicht 2250 g/mol, OH-Zahl 25 mg KOH/g (Bayer MaterialScience AG, Leverkusen, DE) |

### Beispiel 1:

Dieses Beispiel beschreibt die Herstellung einer erfindungsgemäßen Polyurethanharnstofflösung.

198,6 g Desmophen C 2200, 23,0 g LB 25 und 47,8 g 4,4'-Bis(isocyanatocyclohexyl)methan (H₁₂MDI) wurden bei 110 °C in der Schmelze bis zu einem konstanten NCO-Gehalt von 2,4 % umgesetzt. Man ließ abkühlen und verdünnte mit 350,0 g Toluol und 200 g iso-Propanol. Bei Raumtemperatur wurden eine Lösung von 12,5 g Isophorondiamin in 95,0 g 1-Methoxypropanol-2 zugegeben. Nach beendetem Aufbau des Molgewichtes und Erreichen des gewünschten Viskositätsbereiches (Überprüfung durch Messen der Viskosität einer gezogenen Probe mit dem oben zitierten Rheometer) rührte man weitere 4 Stunden, um den restlichen Isocyanatgehalt mit iso-Propanol zu blockieren. Man erhielt 927 g einer 30,4%igen Polyurethanharnstofflösung in Toluol/iso-Propanol/1-Methoxypropanol-2 mit einer Viskosität von 19600 mPas bei 23 °C.

### Beispiel 2:

Dieses Beispiel beschreibt die Herstellung einer erfindungsgemäßen Polyurethanharnstofflösung.

195,4 g Desmophen C 2200, 30,0 g LB 25 und 47,8 g 4,4'-Bis(isocyanatocyclohexyl)methan (H₁₂MDI) wurden bei 110 °C bis zu einem konstanten NCO-Gehalt von 2,3 % umgesetzt. Man ließ abkühlen und verdünnte mit 350,0 g Toluol und 200 g iso-Propanol. Bei Raumtemperatur wurden eine Lösung von 12,7 g Isophorondiamin in 94,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 930 g einer 30,7%igen Polyurethanharnstofflösung in Toluol/iso-Propanol/1-Methoxypropanol-2 mit einer Viskosität von 38600 mPas bei 23 °C.

### Beispiel 3:

Dieses Beispiel beschreibt die Herstellung einer erfindungsgemäßen Polyurethanharnstofflösung.

195,4 g Desmophen XP 2613, 30,0 g LB 25 und 47,8 g 4,4'-Bis(isocyanatocyclohexyl)methan (H₁₂MDI) wurden bei 110 °C bis zu einem konstanten NCO-Gehalt von 2,4 % umgesetzt. Man ließ abkühlen und verdünnte mit 350,0 g Toluol und 200 g iso-Propanol. Bei Raumtemperatur wurden eine Lösung von 12,7 g Isophorondiamin in 95,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 931 g einer 30,7%igen Polyurethanharnstofflösung in Toluol/iso-Propanol/1-Methoxypropanol-2 mit einer Viskosität von 26500 mPas bei 23 °C**.**

### Beispiel 4:

Dieses Beispiel beschreibt die Herstellung einer erfindungsgemäßen Polyurethanharnstofflösung.

198,6 g Desmophen C 1200, 23,0 g LB 25 und 47,8 g 4,4'-Bis(isocyanatocyclohexyl)methan (H₁₂MDI) wurden bei 110 °C bis zu einem konstanten NCO-Gehalt von 2,4 % umgesetzt. Man ließ abkühlen und verdünnte mit 350,0 g Toluol und 200 g iso-Propanol. Bei Raumtemperatur wurden eine Lösung von 13,2 g Isophorondiamin in 100,0 g 1-Methoxypropanol-2 zugegeben. Nach beendetem Aufbau des Molgewichtes und Erreichen des gewünschten Viskositätsbereiches rührte man weitere 4 Stunden, um den restlichen

Isocyanatgehalt mit iso-Propanol zu blockieren. Man erhielt 933 g einer 30,3%igen Polyurethanharnstofflösung in Toluol/iso-Propanol/1-Methoxypropanol-2 mit einer Viskosität von 17800 mPas bei 23 °C.

### Beispiel 5:

Dieses Beispiel beschreibt die Herstellung einer erfindungsgemäßen Polyurethanharnstofflösung.

195,4 g Desmophen C 1200, 30,0 g LB 25 und 47,8 g 4,4'-Bis(isocyanatocyclohexyl)methan (H₁₂MDI) wurden bei 110 °C bis zu einem konstanten NCO-Gehalt von 2,4 % umgesetzt. Man ließ abkühlen und verdünnte mit 350,0 g Toluol und 200 g iso-Propanol. Bei Raumtemperatur wurden eine Lösung von 11,8 g Isophorondiamin in 94,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 931 g einer 30,7%igen Polyurethanharnstofflösung in Toluol/iso-Propanol/1-Methoxypropanol-2 mit einer Viskosität von 23700 mPas bei 23 °C.

### Beispiel 6:

Dieses Beispiel beschreibt die Herstellung einer Polyurethanharnstofflösung als Vergleichsprodukt zu dem erfindungsgemäßen Beispiel 1. Das Desmophen C2200 wird durch das PolyTHF 2000 ausgetauscht.

194,0 g PolyTHF 2000, 22,6 g LB 25 und 47,8 g 4,4'-Bis(isocyanatocyclohexyl)methan (H₁₂MDI) wurden bei 110 °C bis zu einem konstanten NCO-Gehalt von 2,3 % umgesetzt. Man ließ abkühlen und verdünnte mit 350,0 g Toluol und 200 g iso-Propanol. Bei Raumtemperatur wurden eine Lösung von 12,1 g Isophorondiamin in 89,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 916 g einer 30,2%igen Polyurethanharnstofflösung in Toluol/iso-Propanol/1-Methoxypropanol-2 mit einer Viskosität von 15200 mPas bei 23 °C.

### Beispiel 7:

Dieses Beispiel beschreibt die Herstellung einer Polyurethanharnstofflösung als Vergleichsprodukt zu dem erfindungsgemäßen Beispiel 2. Das Desmophen C2200 wird durch das PolyTHF 2000 ausgetauscht.

190,6 g PolyTHF 2000, 30,0 g LB 25 und 47,8 g 4,4'-Bis(isocyanatocyclohexyl)methan (H₁₂MDI) wurden bei 110 °C bis zu einem konstanten NCO-Gehalt von 2,3 % umgesetzt. Man ließ abkühlen und verdünnte mit 350,0 g Toluol und 200 g iso-Propanol. Bei Raumtemperatur wurden eine Lösung von 12,1 g Isophorondiamin in 89,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 919 g einer 30,5%igen Polyurethanharnstofflösung in Toluol/iso-Propanol/1-Methoxypropanol-2 mit einer Viskosität von 21000 mPas bei 23 °C.

### Beispiel 8: Herstellung der Beschichtungen und Messung des statischen Kontaktwinkels

Die Beschichtungen zur Messung des statischen Kontaktwinkels wurden auf Glasobjektträger der Größe 25x75 mm mit Hilfe eines Spincoaters (RC5 Gyrset 5, Karl Süss, Garching, Deutschland) hergestellt. Ein Objektträger wurde hierzu auf dem Probenteller des Spincoaters eingespannt und mit ca. 2,5 - 3 g organischer 15%iger Polyurethanlösung homogen bedeckt Alle organischen Polyurethanlösungen wurden mit einem Lösungsmittelgemisch aus 65 Gew% Toluol und 35 Gew% iso-Propanol auf einen Polymergehalt von 15 Gew% verdünnt. Durch Rotation des Probentellers für 20 sec bei 1300 Umdrehungen pro Minute erhielt man eine homogene Beschichtung, die 1 h bei 100 °C und danach 24 h bei 50 °C getrocknet wurde. Die erhaltenen beschichteten Objektträger wurden direkt einer Kontaktwinkelmessung unterzogen.

Von den erhaltenen Beschichtungen auf den Objektträgern wurde eine statische Kontaktwinkelmessung durchgeführt. Mittels des Video- Randwinkelmessgerätes OCA20 der Firma Dataphysics mit rechnergesteuerten Spritzen wurde auf das Muster 10 Tropfen Milliporewasser aufgesetzt und deren statischer Benetzungsrandwinkel gemessen. Zuvor wurde mittels eines Antistatikföns die statische Aufladung (falls vorhanden) auf der Probenoberfläche entfernt.

**Tabelle 1: Statische Kontaktwinkelmessungen**

| PU-FILM | Kontaktwinkel [°] |
|---|---|
| Beispiel 1 | 32 |
| Beispiel 2 | 21 |
| Beispiel 3 | 38 |
| Beispiel 4 | 25 |
| Beispiel 5 | 25 |
| Beispiel 6 (Vergleichsbeispiel) | 83 |
| Beispiel 7 (Vergleichsbeispiel) | 82 |

Wie Tabelle 1 zeigt, ergeben die polycarbonathaltigen Beschichtungen der Beispiele 1-5 äußerst hydrophile Beschichtungen mit statischen Kontaktwinkeln ≤ 40 °. Dagegen sind die PolyTHF-haltigen Beschichtungen 7-9 wesentlich unpolarer, obwohl die Zusammensetzung dieser Beschichtungen ansonsten mit denen der Beispiele 1 und 2 identisch sind.

### Beispiel 9:

Dieses Vergleichsbeispiel beschreibt die Synthese eines Polyurethanharnstoffpolymers, welches anstelle des gemischten monofunktionellen Polyethylen-Polypropylenoxidalkohols LB 25 den gleichen molaren Anteil eines reinen monofunktionellen Polyethylenoxidalkohols enthält. Das Polymer ist mit demjenigen des Beispiels 1 identisch, nur dass es eine andere terminale Gruppe enthält. Die Synthese in Toluol und Alkoholen wie in den Beispielen 1 bis 7 beschrieben funktioniert bei Verwendung dieses Alkohols nicht. Daher wird die Synthese in reinem Dimethylformamid (DMF) durchgeführt.

198,6 g Desmophen C 2200, 20,4 g Polyethylenglykol-2000-monomethylether (Quelle: Fluka, Artikel-Nr. 81321) und 47,8 g 4,4'-Bis(isocyanatocyclohexyl)methan (H₁₂MDI) wurden bei 110 °C in der Schmelze bis zu einem konstanten NCO-Gehalt von 2,4 % umgesetzt. Man ließ abkühlen und verdünnte mit 550 g DMF. Bei Raumtemperatur wurden eine Lösung von 10,5 g Isophorondiamin in 100 g DMF zugegeben. Nach beendetem Aufbau des Molgewichtes und Erreichen des gewünschten Viskositätsbereiches (Überprüfung durch Messen der Viskosität einer gezogenen Probe mit dem oben zitierten Rheometer) gab man 1,0 g n-Butylamin hinzu, um den verbliebenen geringen Isocyanatgehalt zu blockieren. Man erhielt 927 g einer 29,8%igen Polyurethanharnstofflösung in Dimethylformamid mit einer Viskosität von 22700 mPas bei 23 °C.

### Beispiel 10:

Dieses Beispiel beschreibt die Synthese eines erfindungsgemäßen Polyurethanharnstoffpolymers in DMF als Lösungsmittel. Das Polymer ist mit demjenigen des Beispiels 1 identisch, wurde aber deshalb in DMF hergestellt, um seine physikalischen Eigenschaften mit dem Polymer des Beispiels 9 vergleichen zu können.

198,6 g Desmophen C 2200, 23,0 g LB 25 und 47,8 g 4,4'-Bis(isocyanatocyclohexyl)methan (H₁₂MDI) wurden bei 110 °C in der Schmelze bis zu einem konstanten NCO-Gehalt von 2,4 % umgesetzt. Man ließ abkühlen und verdünnte mit 550 g DMF. Bei Raumtemperatur wurden eine Lösung von 10,5 g Isophorondiamin in 100 g 1-Methoxypropanol-2 zugegeben. Nach beendetem Aufbau des Molgewichtes und Erreichen des gewünschten Viskositätsbereiches (Überprüfung durch Messen der Viskosität einer gezogenen Probe mit dem oben zitierten Rheometer) gab man 0,5 g n-Butylamin zu, um den geringen verbliebenen Isocyanatgehalt zu blockieren. Man erhielt 930 g einer 30,6%igen Polyurethanharnstofflösung in DMF mit einer Viskosität von 16800 mPas bei 23 °C.

### Beispiel 11:

Wie in Beispiel 8 beschrieben, wurden mit den Polyurethanlösungen der Beispiele 9 und 10 Filme auf Glas hergestellt und statische Kontaktwinkel gemessen.

**Tabelle 2: Statische Kontaktwinkel in Abhängigkeit der verwendeten monofunktionellen Polyether**

| PU-FILM | Kontaktwinkel[°] |
|---|---|
| Beispiel 9 (Vergleichsbeispiel) | 55 |
| Beispiel 10 (erfindungsgemäßes Beispiel) | 36 |

Der mit dem gemischten (Polyethylenoxid/Polypropylenoxid) monofunktionellen Polyetheralkohol hergestellte Film des Beispiels 10 zeigt mit 36 ° einen deutlich niedrigeren statischen Kontaktwinkel als derjenige Film des Beispiels 9 (55 °), der reine Polyethylenoxideinheiten enthält.

### Beispiel 12:

Dieses Beispiel beschreibt die Synthese eines erfindungsgemäßen Polyurethans in organischer Lösung. Dieses Produkt wurde mit dem entsprechend in wässriger Dispersion hergestellten Polyurethans des Beispiels 13 verglichen (siehe Beispiel 14).

277,2 g Desmophen C 2200, 33,1 g LB 25, 6,7 g Neopentylglykol, 71,3 g 4,4'-Bis(isocyanatocyclohexyl)methan (H₁₂MDI) und 11,9 g Isophorondiisocyanat wurden bei 110 °C bis zu einem konstanten NCO-Gehalt von 2,4 % umgesetzt. Man ließ abkühlen und verdünnte mit 500,0 g Toluol und 350,0 g iso-Propanol. Bei Raumtemperatur wurden eine Lösung von 6,2 g Isophorondiamin in 186,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 1442 g einer 28,6%igen Polyurethanharnstofflösung in Toluol/iso-Propanol/1-Methoxypropanol-2 mit einer Viskosität von 17500 mPas bei 23 °C.

### Beispiel 13:

Dieses Beispiel beschreibt die Synthese des Polyurethans des Beispiels 12 in wässriger Dispersion. Es besteht aus demselben Polymer wie in Beispiel 12 beschrieben. Beide Polymere werden in Beispiel 14 miteinander verglichen.

277,2 g Desmophen C 2200, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondüsocyanat. Man erwärmte auf 110 °C, bis ein konstanter NCO-Wert von 2,4 % erreicht wurde. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 40,7 % und einer mittleren Teilchengröße von 136 nm erhalten. Der pH-Wert dieser Dispersion betrug 6,7.

### Beispiel 14:

Die beiden Beschichtungen des Beispiels 12 und 13 wurden auf Trennpapier mit einem 200 µm-Rakel aufgetragen. Die Beschichtung des Beispiels 12 wurde in unverdünnter Form aufgetragen, die wässrige Dispersion wurde vor der Filmherstellung mit 2 Gew% eines Verdickers (Borchi Gel A LA, Fa. Borchers, Langenfeld, Deutschland) versetzt und 30 min bei RT durch Rühren homogenisiert. Die feuchten Filme wurden 15 min bei 100 °C getrocknet.

Gemessen werden Zugfestigkeit und Bruchdehnung im trockenen Zustand und nach 24 h Wässern der Filme. Die Untersuchungen wurden gemäß DIN 53504 durchgeführt.

**Tabelle 3: Vergleich der Zugfestigkeitsergebnisse für Polyurethan aus organischer Lösung und wässriger Dispersion**

| Film | Bruchspannung (N/mm²) trockener Film | Bruchspannung (N/mm²) 24 h Wasser | Bruchdehnung (%) trockner Film | *Bruchdehnung (%) 24 h Wasser* |
|---|---|---|---|---|
| Beispiel 12 | 25,3 | 24,9 | 700 | *700* |
| *Beispiel 13* | *24,8* | *18,3* | *550* | *450* |

Die Ergebnisse der Tabelle zeigen, dass die Bruchspannung der getrockneten Filme für beide Polyurethane, unabhängig von der Herstellung als Lösung oder als wässriger Dispersion, im Rahmen der experimentellen Genauigkeit übereinstimmen. Der aus organischer Lösung hergestellte Film des Beispiels 12 besitzt aber eine höhere Elastizität (700 % Bruchdehnung im Vergleich zu 550 % für das Polymer aus wässriger Dispersion). Darüber hinaus verändern sich Bruchspannung und Bruchdehnung für den aus organischer Lösung hergestellten Film im Rahmen der Messgenauigkeit nicht, während Bruchspannung und Bruchdehnung des aus wässriger Dispersion hergestellten Films deutlich abnehmen.

## Patentansprüche

1. Verwendung einer lösung enthaltend mindestens einen Polyurethanharnstoff, wobei der Polyurethanharnstoff mit einer Copolymereinheit aus Polyethylenoxid und Polypropylenoxid terminiert ist, zur Herstellung einer Beschichtung für ein medizinisches Gerät.

2. Verwendung nach Anspruch 1, **dadurch gekennzeichnet, dass** der Polyurethanharnstoff Einheiten aufweist, welche auf mindestens ein Hydroxylgruppen enthaltendes Polycarbonat zurückgehen.

3. Verwendung nach Anspruch 1 oder 2, **dadurch gekennzeichnet, dass** der Polyurethanharnstoff Einheiten aufweist, welche auf mindestens ein aliphatisches, cycloaliphatisches oder aromatisches Isocyanat zurückgehen.

4. Verwendung nach einem der Ansprüche 1 bis 3, **dadurch gekennzeichnet, dass** der Polyurethanharnstoff Einheiten aufweist, die auf mindestens ein Diamin oder Aminalkohol zurückgehen.

5. Verwendung nach einem der Ansprüche 1 bis 4, **dadurch gekennzeichnet, dass** die Beschichtung einen Polyurethanharnstoff aufweist, welcher aufgebaut ist aus
a) mindestens einem Polycarbonatpolyol mit einem mittleren Molgewicht zwischen 400 g/mol und 6000 g/mol und einer Hydroxylfunktionalität von 1,7 bis 2,3 oder aus Mischungen solcher Polycarbonatpolyole;
b) mindestens einem aliphatischen, cycloaliphatischen oder aromatischen Polyisocyanat oder Mischungen solcher Polyisocyanate in einer Menge pro Mol des Polyearbonatpolyols von 1,0 bis 3,5 mol;
c) mindestens einem aliphatischen oder cycloaliphatischen Diamin oder mindestens ein Aminoalkohol als so genannte Kettenverlängerer oder Mischungen solcher Verbindungen in einer Menge pro Mol des Polycarbonatpolyols von 0,05 bis 3,0 mol;
d) mindestens einem monofunktionellen gemischten Polyoxyalkylenether aus Polyethylenoxid und Polypropylenoxid oder einer Mischung solcher Polyether mit einem mittleren Molgewicht zwischen 500 g/mol und 5000 g/mol in einer Menge pro Mol des Polycarbonatpolyols von 0,1 bis 1,5 mol;
e) gegebenenfalls einem oder mehreren kurzkettigen aliphatischen Polyolen mit einem Molgewicht zwischen 62 g/mol und 500 g/mol in einer Menge pro Mol des Polycarbonatpolyols von 0,1 bis 1,0 mol; und
f) gegebenenfalls amin- oder OH haltige Bausteine, die sich an den Polymerkettenenden befinden und diese abschließen.

6. Verwendung nach Anspruch 1, **dadurch gekennzeichnet, dass** die Beschichtung ausgehend von einer Lösung des Polyurethanharnstoffes durch Rakeln, Drucken, Transferbeschichten, Sprühen, Spincoating oder Tauchen auf dem medizinischen Gerät aufgebracht wird.

## Claims

1. Use of a solution comprising at least one polyurethaneurea, the polyurethaneurea being terminated with a copolymer unit comprising polyethylene oxide and polypropylene oxide, for producing a coating for a medical device.

2. Use according to Claim 1, **characterized in that** the polyurethaneurea comprises units which originate from at least one hydroxyl-containing polycarbonate.

3. Use according to Claim 1 or 2, **characterized in that** the polyurethaneurea comprises units which originate from at least one aliphatic, cycloaliphatic or aromatic isocyanate.

4. Use according to one of Claims 1 to 3, **characterized in that** the polyurethaneurea comprises units which originate from at least one diamine or amine alcohol.

5. Use according to one of Claims 1 to 4, **characterized in that** the coating comprises a polyurethaneurea which is synthesized from
a) at least one polycarbonate polyol having an average molar weight between 400 g/mol and 6000 g/mol and a hydroxyl functionality of 1.7 to 2.3, or mixtures of such polycarbonate polyols;
b) at least one aliphatic, cycloaliphatic or aromatic polyisocyanate or mixtures of such polyisocyanates in an amount per mole of the polycarbonate polyol of 1.0 to 3.5 mol;
c) at least one aliphatic or cycloaliphatic diamine or at least one amino alcohol, as so-called chain extenders, or mixtures of such compounds in an amount per mole of the polycarbonate polyol of 0.05 to 3.0 mol;
d) at least one monofunctional mixed polyoxyalkylene ether comprising polyethylene oxide and polypropylene oxide or a mixture of such polyethers, having an average molar weight between 500 g/mol and 5000 g/mol, in an amount per mole of the polycarbonate polyol of 0.1 to 1.5 mol;
e) if desired, one or more short-chain aliphatic polyols having a molar weight between 62 g/mol and 500 g/mol, in an amount per mole of the polycarbonate polyol of 0.1 to 1.0 mol; and
f) if desired, amine- or OH-containing units which are located on, and cap, the polymer chain ends.

6. Use according to Claim 1, **characterized in that** the coating starting from a solution of the polyurethaneurea is applied to the medical device by knifecoating, printing, transfer coating, spraying, spin coating or dipping.

## Revendications

1. Utilisation d'une solution contenant au moins une polyuréthane-urée, la polyuréthane-urée étant terminée avec une unité copolymère d'oxyde de polyéthylène et d'oxyde de polypropylène, pour la fabrication d'un revêtement pour un appareil médical.

2. Utilisation selon la revendication 1, **caractérisée en ce que** la polyuréthane-urée comprend des unités qui remontent à au moins un polycarbonate contenant des groupes hydroxyle.

3. Utilisation selon la revendication 1 ou 2, **caractérisée en ce que** la polyuréthane-urée comprend des unités qui remontent à au moins un isocyanate aliphatique, cycloaliphatique ou aromatique.

4. Utilisation selon l'une quelconque des revendications 1 à 3, **caractérisée en ce que** la polyuréthane-urée comprend des unités qui remontent à au moins une diamine ou un aminoalcool.

5. Utilisation selon l'une quelconque des revendications 1 à 4, **caractérisée en ce que** le revêtement comprend une polyuréthane-urée qui est constituée par
a) au moins un polycarbonate-polyol ayant un poids moléculaire moyen compris entre 400 g/mol et 6 000 g/mol et une fonctionnalité hydroxyle de 1,7 à 2,3 ou de mélanges de tels polycarbonate-polyols ;
b) au moins un polyisocyanate aliphatique, cycloaliphatique ou aromatique ou des mélanges de tels polyisocyanates en une quantité par mole du polycarbonate-polyol de 1,0 à 3,5 moles ;
c) au moins une diamine aliphatique ou cycloaliphatique ou au moins un aminoalcool en tant qu'agent dit allongeur de chaîne ou des mélanges de tels composés en une quantité par mole du polycarbonate-polyol de 0,05 à 3,0 moles ;
d) au moins un éther de polyoxyalkylène mixte monofonctionnel d'oxyde de polyéthylène et d'oxyde de polypropylène ou un mélange de tels polyéthers ayant un poids moléculaire moyen compris entre 500 g/mol et 5 000 g/mol en une quantité par mole du polycarbonate-polyol de 0,1 à 1,5 mole ;
e) éventuellement un ou plusieurs polyols aliphatiques à chaîne courte ayant un poids moléculaire compris entre 62 g/mol et 500 g/mol en une quantité par mole du polycarbonate-polyol de 0,1 à 1,0 mole ; et
f) éventuellement des constituants contenant des groupes amino ou OH, qui se trouvent sur les extrémités des chaînes polymères et terminent celles-ci.

6. Utilisation selon la revendication 1, **caractérisée en ce que** le revêtement est appliqué sur l'appareil médical à partir d'une solution de la polyuréthane-urée par raclage, impression, revêtement par transfert, pulvérisation, dépôt à la tournette ou immersion.
